# EUROPEAN PATENT APPLICATION

(11) **EP 3 730 189 A1**
(43) Date of publication of application: **28.10.2020**
(21) Application number: 19193258.1
(22) Date of filing: 23.08.2019
(51) Int. Cl.: A62B 21/00, A62B 7/08, A61M 16/10, C01B 13/02

(54) **AN OXYGEN GENERATOR**

(30) Priority: 26.04.2019 IN 201941016754
(71) Applicant: O2-Matic Products Private Limited, Bangalore 560048 (IN)
(72) Inventor: SACHIDANADAM, John Paul Thambusami Joy, 560048 Bangalore (IN)
(74) Representative: Caldon, Giuliano

(57) **Abstract**

An oxygen generator is provided. The oxygen generator includes a generator case comprising at least two hollow chambers, at least two cartridges housed within the corresponding at least two hollow chambers. Each of the at least two cartridges include a water chamber, a chemical chamber, a holding means mechanically coupled to an interior top portion of the at least two cartridges, a triggering means mechanically held in place by the holding means. The triggering means include a top seal configured to unlock a bottom seal upon twisting a trigger connector, a trigger valve, a hollow seal tube located along a central axis of the corresponding at least two cartridges, the bottom seal configured to allow a pre-defined amount of water to enter the chemical chamber via a filter. The hollow seal tube is configured to allow flow of oxygen generated in the chemical chamber.

## Description

### FIELD OF INVENTION

Embodiments of a present invention relate to an oxygen generator, and more particularly to an oxygen generator.

### BACKGROUND

Oxygen is a type of a colourless and an odourless gas which majorly supports life on earth. In certain situation, oxygen is stored in tanks and provided to people having certain breathing issues or undergoing operation so as to maintain a desired level of oxygen in breathing air. In certain other cases, tanks storing oxygen under pre-defined pressure referred to as oxygen cylinders is also used by people in other situations such as travel in space shuttle, scuba diving, mountaineering and the like for maintaining required supply of oxygen. However, such oxygen cylinders are usually composed of metals and are heavy in weight and big in size which creates a problem for transportation or porting of such cylinders. Bulky oxygen cylinders make it very difficult to carry them around. Also, refilling of oxygen into the cylinder is a lengthy and expensive process. Thereby oxygen generators are being used to enable easy porting of the oxygen.

In one such approach, an oxygen generator is used to generate and store the oxygen upon combining a number of chemicals together in a vessel at a required pressure. Consequently, the oxygen generator generates the oxygen at any instant of time. However, rate of flow of generation of oxygen by the oxygen generator cannot be maintained and also duration for which the oxygen generator can generate oxygen is limited or short. Such limitation may not produce a required quantity of oxygen for a desired duration of time. Also, a user needs to manually mix number of chemicals in order to generate the oxygen. Such human interventions can lead to inaccuracy in ratio of the number of chemicals, thereby making the oxygen generator less efficient and less accurate.

Hence, there is a need for an improved oxygen generator to address the aforementioned issues.

### BRIEF DESCRIPTION

In accordance with one embodiment of the disclosure, an oxygen generator is provided. The oxygen generator includes a generator case. The generator case includes at least two hollow chambers. The oxygen generator also includes at least two cartridges housed within the corresponding at least two hollow chambers. Each of the at least two cartridges include a water chamber configured to store a pre-defined amount of water. Each of the At least two cartridges also include a chemical chamber mechanically coupled to the water chamber. The chemical chamber is configured to store one or more chemicals in a pre-defined ratio. The water chamber is placed above the chemical chamber and is separated by a disc and is pneumatically sealed. Each of the At least two cartridges also include a holding means mechanically coupled to an interior top portion of the corresponding at least two cartridges. Each of the At least two cartridges also include a triggering means mechanically held in place by the holding means. The triggering means include a top seal configured to unlock a bottom seal upon twisting a trigger connector to a pre-defined angle. The triggering means also include a trigger valve operatively coupled to the top seal. The trigger valve is configured to enable a process of generation of oxygen. The triggering means also includes a hollow seal tube located along a central axis of the corresponding at least two cartridges. The triggering means also includes the bottom seal operatively coupled at a bottom of the hollow seal tube. The bottom seal is configured to allow the pre-defined amount of water to enter the chemical chamber via a filter. The filter is fixed to the disc within the chemical chamber. The hollow seal tube is configured to allow flow of oxygen generated from chemical chamber to the water chamber. The hollow seal tube is also configured to allow flow of oxygen generated in the chemical chamber upon reaction of the pre-defined amount of water with the one or more chemicals to exit from the water chamber. The at least two cartridges are placed in the corresponding at least two hollow chambers of the generator case.

To further clarify the advantages and features of the present disclosure, a more particular description of the disclosure will follow by reference to specific embodiments thereof, which are illustrated in the appended figures. It is to be appreciated that these figures depict only typical embodiments of the disclosure and are therefore not to be considered limiting in scope. The disclosure will be described and explained with additional specificity and detail with the appended figures.

### BRIEF DESCRIPTION OF THE DRAWINGS

The disclosure will be described and explained with additional specificity and detail with the accompanying figures in which:
FIG. 1 is a schematic cross-sectional view of an oxygen generator in accordance with an embodiment of the present disclosure;
FIG. 2 is a schematic representation of an embodiment of an external view of the oxygen generator of FIG. 1 in accordance with an embodiment of the present disclosure;
FIG. 3 is a schematic representation of another embodiment of an external view of the oxygen generator of FIG. 1 in accordance with an embodiment of the present disclosure;
FIG. 4 is a schematic representation of an embodiment representing an internal view of the oxygen generator of FIG. 1 in accordance with an embodiment of the present disclosure;
FIG. 5 is a schematic representation of an oxygen generator with a humidifier means in accordance with an embodiment of the present disclosure; and
FIG. 6 is a schematic representation of an embodiment representing housing of the oxygen generator with the humidifier means of FIG. 5 in accordance with an embodiment of the present disclosure.

Further, those skilled in the art will appreciate that elements in the figures are illustrated for simplicity and may not have necessarily been drawn to scale. Furthermore, in terms of the construction of the device, one or more components of the device may have been represented in the figures by conventional symbols, and the figures may show only those specific details that are pertinent to understanding the embodiments of the present disclosure so as not to obscure the figures with details that will be readily apparent to those skilled in the art having the benefit of the description herein.

### DETAILED DESCRIPTION

For the purpose of promoting an understanding of the principles of the disclosure, reference will now be made to the embodiment illustrated in the figures and specific language will be used to describe them. It will nevertheless be understood that no limitation of the scope of the disclosure is thereby intended. Such alterations and further modifications in the illustrated system, and such further applications of the principles of the disclosure as would normally occur to those skilled in the art are to be construed as being within the scope of the present disclosure.

The terms "comprises", "comprising", or any other variations thereof, are intended to cover a non-exclusive inclusion, such that a process or method that comprises a list of steps does not include only those steps but may include other steps not expressly listed or inherent to such a process or method. Similarly, one or more devices or sub-systems or elements or structures or components preceded by "comprises... a" does not, without more constraints, preclude the existence of other devices, sub-systems, elements, structures, components, additional devices, additional sub-systems, additional elements, additional structures or additional components. Appearances of the phrase "in an embodiment", "in another embodiment" and similar language throughout this specification may, but not necessarily do, all refer to the same embodiment.

Unless otherwise defined, all technical and scientific terms used herein have the same meaning as commonly understood by those skilled in the art to which this disclosure belongs. The system, methods, and examples provided herein are only illustrative and not intended to be limiting.

In the following specification and the claims, reference will be made to a number of terms, which shall be defined to have the following meanings. The singular forms "a", "an", and "the" include plural references unless the context clearly dictates otherwise.

Embodiments of the present disclosure relate to an oxygen generator. The oxygen generator includes a generator case. The generator case includes at least two hollow chambers. The oxygen generator also includes at least two cartridges housed within the corresponding at least two hollow chambers. Each of the at least two cartridges include a water chamber configured to store a pre-defined amount of water. Each of the At least two cartridges also include a chemical chamber mechanically coupled to the water chamber. The chemical chamber is configured to store one or more chemicals in a pre-defined ratio. The water chamber is placed above the chemical chamber and is separated by a disc and is pneumatically sealed. Each of the At least two cartridges also include a holding means mechanically coupled to an interior top portion of the corresponding at least two cartridges. Each of the At least two cartridges also include a triggering means mechanically held in place by the holding means. The triggering means include a top seal configured to unlock a bottom seal upon twisting a trigger connector to a pre-defined angle. The triggering means also include a trigger valve operatively coupled to the top seal. The trigger valve is configured to enable a process of generation of oxygen. The triggering means also includes a hollow seal tube located along a central axis of the corresponding at least two cartridges. The triggering means also includes the bottom seal operatively coupled at a bottom of the hollow seal tube. The bottom seal is configured to allow the pre-defined amount of water to enter the chemical chamber via a filter. The filter is fixed to the disc within the chemical chamber. The hollow seal tube is configured to allow flow of oxygen generated from chemical chamber to the water chamber. The hollow seal tube is also configured to allow flow of oxygen generated in the chemical chamber upon reaction of the pre-defined amount of water with the one or more chemicals to exit from the water chamber. The at least two cartridges are placed in the corresponding at least two hollow chambers of the generator case.

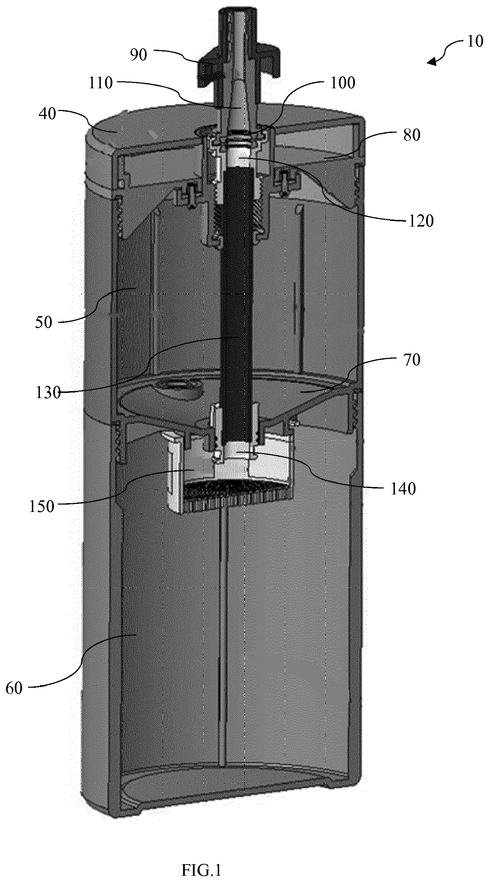

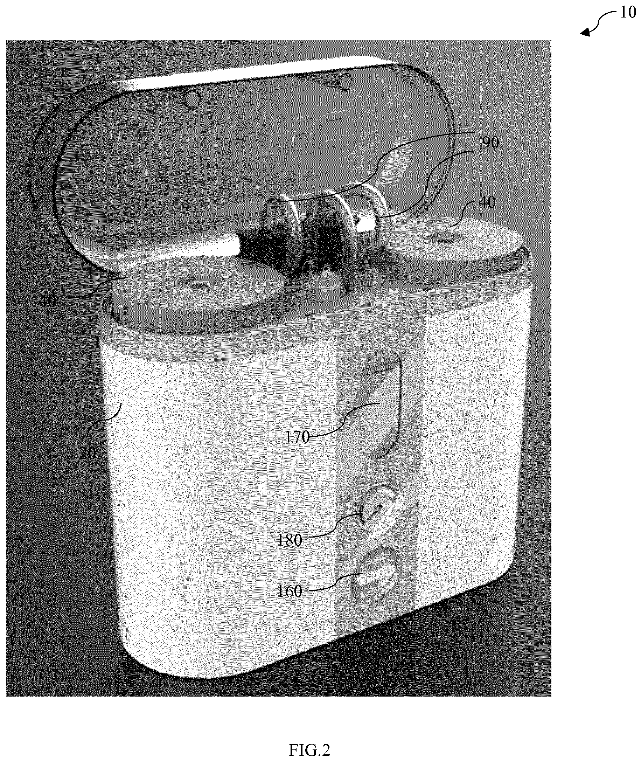

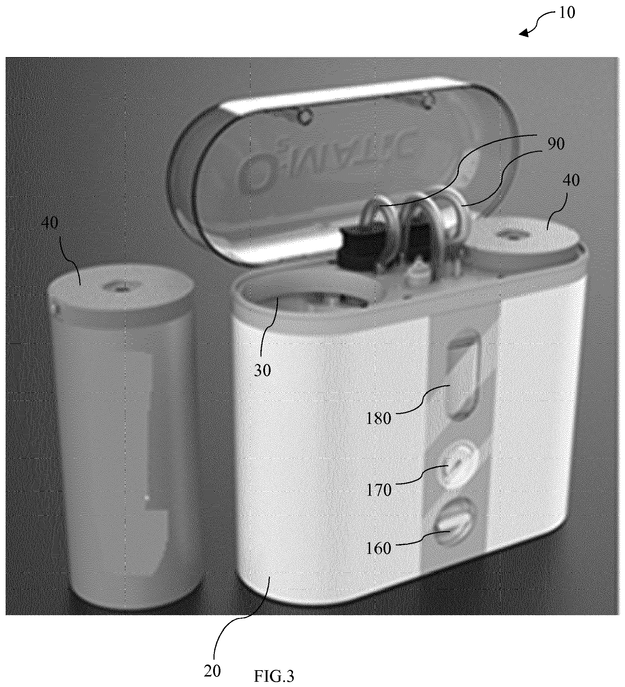

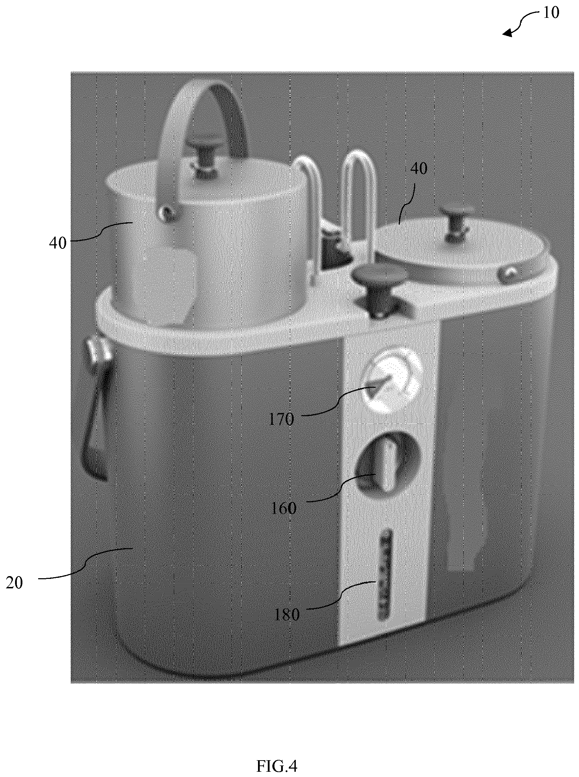

FIG. 1 is a schematic cross-sectional view of an oxygen generator (10) in accordance with an embodiment of the present disclosure. As used herein, the term "oxygen generator" is defined as a medical device that generates oxygen as a result of a reaction of one or more elements. The oxygen generator (10) includes a generator case (20) (as shown in FIG. 2, FIG. 3 and FIG. 4) comprising at least two hollow chambers (30). In one embodiment, each of the at least two hollow chambers (30) may correspond to a hollow cylindrical chamber. The at least two hollow cylindrical chambers may be separated by a solid material organised in a pre-defined shape.

The oxygen generator (10) also includes at least two cartridges (40) housed within the corresponding at least two hollow chambers (30). As used herein, the term "cartridge" is defined as a hollow container for holding material used for an insertion into a mechanism. In one exemplary embodiment, each of the at least cartridges (40) are coupled with a handle which is configured to operate the at least two cartridges (40). In such embodiment, operation of the at least two cartridges (40) may include loading and unloading the at least two cartridges (40) into the corresponding at least two hollow chambers (30). Furthermore, each of the at least two cartridges (40) include a water chamber (50). The water chamber (50) is configured to store a pre-defined amount of water.

Each of the at least two cartridges (40) also include a chemical chamber (60) mechanically coupled to the water chamber (50). The chemical chamber (60) is configured to store one or more chemicals in a pre-defined ratio. In one embodiment, the one or more chemicals includes at least one of sodium percarbonate, potassium superoxide, peroxide species (hydrogen peroxide), urea-hydrogen peroxide and percarbamide peroxide. In one exemplary embodiment, the one or more chemicals may be in a form of one of a granular powder, pellets, powder in a pouch and candle stick.

Furthermore, the water chamber (50) is placed above the chemical chamber (60) and is separated by a disc (70) and is pneumatically sealed. Each of the at least two cartridges (40) also include a holding means (80) mechanically coupled to an interior top portion of the corresponding at least two cartridges (40).

Each of the at least two cartridges (40) also include a triggering means (90) mechanically held in place by the holding means (80). As used herein, the term "triggering means" is defined as a means used to trigger or cause a particular reaction based on a particular action. The triggering means (90) includes a top seal (100) configured to unlock a bottom seal (140) upon twisting a trigger connector (110) to a pre-defined angle. In one embodiment, the top seal (100) may be twisted manually by a user to the pre-defined angle. In such embodiment, the pre-defined angle may be 90 degrees. In one embodiment, the trigger connector (110) may be placed on a top surface of the generator case (20). The trigger connector (110) may be configured to keep the connection of the at least two cartridges (40) in place during the generation of oxygen.

Also, the triggering means (90) includes a trigger valve (120) operatively coupled to the top seal (100). The trigger valve (120) is configured to enable a process of generation of oxygen. More specifically, the generation of the oxygen will be initiated upon enabling the one or more chemicals to react with the pre-defined amount of water. In order to enable the reaction, the top seal (100) is first triggered and correspondingly the trigger valve (120) is operated to enable the process of generation of oxygen.

The triggering means (90) also includes a hollow seal tube (130) located along a central axis of the corresponding at least two cartridges (40). As used herein, the term "central axis" is defined as an imaginary straight line passing vertically about a centre of an object. The triggering means (90) also includes the bottom seal (140) operatively coupled at a bottom of the hollow seal tube (130). The bottom seal (140) is configured to allow the pre-defined amount of water to enter the chemical chamber (60) via a filter (150). The filter (150) is fixed to the disc within the chemical chamber (60). More specifically, the pre-defined amount of water stored in the water chamber (50) is made to pass to the chemical chamber (60) on twisting the top seal (100).

Furthermore, the hollow seal tube (130) is configured to allow flow of oxygen generated from chemical chamber (60) to the water chamber (50). More specifically, as the bottom seal (140) is made open, the pre-defined amount of water reacts with the one or more chemicals in the chemical chamber (60) and generate oxygen. Consequently, the generated oxygen is passed through the hollow seal tube

(130). In addition, the hollow seal tube (130) is also configured to allow flow of oxygen generated in the chemical chamber (60) upon reaction of the pre-defined amount of water with the one or more chemicals to exit from the water chamber (50). In one embodiment, the flow of oxygen in the hollow seal tube (130) is uni-directional and may be controlled by the bottom seal (140).

In one exemplary embodiment, the chemical chamber (60) of each of the at least two cartridges (40) may include a mechanical stirrer (not shown in FIG. 1). The mechanical stirrer may be configured to keep the one or more chemicals in the chemical chamber (60) in constant mixing.

In another exemplary embodiment, the oxygen generator (10) may include an oxygen flow control knob (160) operatively coupled to each of the at least two cartridges (40) via the corresponding trigger connector (110). The oxygen flow control knob (160) may be configured to control flow ratio of oxygen generated by each of the at least two cartridges (40). In one specific embodiment, the trigger connector (110) may be configured to prevent disconnection of trigger connector (110) from the corresponding at least two cartridges (40) during the generation of oxygen.

In one specific embodiment, the oxygen generator (10) may further include a humidifier (180) operatively coupled to the oxygen flow control knob (160). The humidifier (180) may be configured to impart humidity to the generated oxygen. As used herein, the term "humidity" is defined as amount of water vapour present in air.

Furthermore, in an exemplary embodiment, the oxygen generator (10) may further include an oxygen level indicator (170) operatively coupled to each of the at least two cartridges (40). The oxygen level indicator (170) may be configured to display oxygen level in each of the at least two cartridges (40). More specifically, the oxygen level indicator (170) may indicate the level of oxygen generated by each of the at least two cartridges (40). In another exemplary embodiment, the oxygen generator (10) may further include an oxygen pressure regulator (not shown in FIG. 1) operatively coupled to each of the at least two cartridges (40). The oxygen pressure regulator may be configured to enable a uniform flow of oxygen. In yet another exemplary embodiment, the oxygen generator (10) may further include a cooling mechanism (not shown in FIG. 1) operatively coupled to each of the at least two cartridges (40). The cooling mechanism may be configured to dissipate heat from each of the at least two cartridges (40). In yet another exemplary embodiment, the oxygen generator (10) may further include at least one storage tank (not shown in FIG. 1) operatively coupled to at least one of the at least two cartridges (40). The at least one storage tank may be configured to enable a uniform flow of oxygen generated in the chemical chamber upon storing the generated oxygen

In one specific embodiment, the oxygen generator (10) may be composed of polymer. In one exemplary embodiment, the at least two cartridges (40) may be sealed within the corresponding at least two hollow chambers (30) by placing and rotating the corresponding at least two cartridges (40) in a clockwise direction. In such embodiment, the at least two cartridges (40) may be sealed within the corresponding at least two hollow chambers (30) by using a bayonet mount mechanism. In such another embodiment, the at least two cartridges (40) may be removed from the corresponding at least two hollow chambers (30) by rotating the at least two cartridges (40) in an anticlockwise direction.

In operation, the triggering connector (110) of the triggering means (90) of a first cartridge of the at least one cartridge (40) is manually twisted to 90 degrees, consequently the top seal (100) and hence the trigger valve (120) of the triggering means (90) of the first cartridge is opened to allow the flow of pre-defined amount of water from the water chamber (50) to enter the chemical chamber (60) via the filter

(150) which is controlled by the bottom seal (140). Further, as the pre-defined amount of water is mixed with the one or more chemicals, oxygen is released as a product. Further, the oxygen generated in the chemical chamber (60) is transferred to thee outlet via the hollow seal tube (130) passing through the water chamber (50). The oxygen generated in the chemical chamber (60) is then transferred to the humidifier (180) to impart humidity to the generated oxygen. Also, the level of oxygen left in the first cartridge is indicated by the oxygen level indicator (170). Furthermore, as the oxygen generated in the first cartridge gets ceased, the triggering connector (110) of the triggering means (90) of a second cartridge of the at least one cartridge (40) is manually twisted to 90 degrees, and the process of generation of oxygen is repeated in the second cartridge as in the first cartridge.

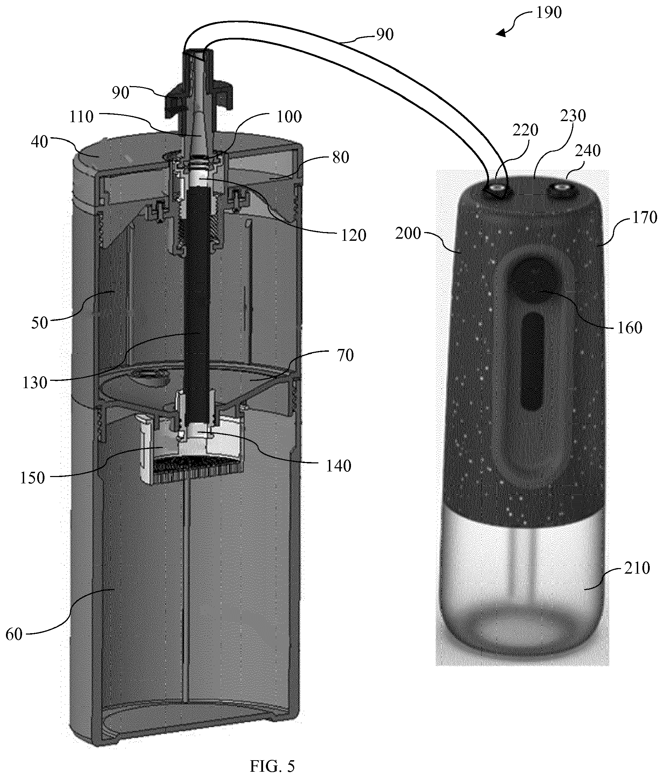

FIG. 5 is a schematic representation of an oxygen generator (190) with a humidifier means (200) in accordance with an embodiment of the present disclosure. The oxygen generator (190) includes at least one cartridge (40). The at least one cartridge (40) includes a water chamber (50). The water chamber (50) is configured to store a pre-defined amount of water. The at least one cartridge (40) also includes a chemical chamber (60) mechanically coupled to the water chamber (50). The chemical chamber (60) is configured to store one or more chemicals in a pre-defined ratio. The water chamber (50) is placed above the chemical chamber (60) and is separated by a disc (70). In addition, the at least one cartridge (40) is pneumatically sealed.

Furthermore, the at least one cartridge (40) also includes a holding means (80) mechanically coupled to an interior top portion of the corresponding at least one cartridge (40). The at least one cartridge (40) also includes a triggering means (90) mechanically held in place by the holding means (80). The triggering means (90) includes a top seal (100). The top seal (100) is configured to unlock a bottom seal (140) upon twisting a trigger connector (110) to a pre-defined angle. The triggering means (90) also includes a trigger valve (120) operatively coupled to the top seal (100). The trigger valve (120) is configured to enable a process of generation of oxygen. The triggering means (90) also includes a hollow seal tube (130) located along a central axis of the corresponding at least one cartridge (40). Also, the bottom seal (140) is operatively coupled at a bottom of the hollow seal tube (130). The bottom seal (140) is operatively coupled at a bottom of the hollow seal tube (130). The bottom seal (140) is configured to allow the pre-defined amount of water to enter the chemical chamber (60) via a filter (150). The filter (150) is fixed to the disc (70) within the chemical chamber (60).

Moreover, the hollow seal tube (130) is configured to allow flow of oxygen generated from chemical chamber (60) to the water chamber (50). The hollow seal tube

(130) is also configured to allow flow of oxygen generated in the chemical chamber (60) upon reaction of the pre-defined amount of water with the one or more chemicals to exit from the water chamber (50).

The oxygen generator (190) also includes the humidifier means (200) operatively coupled to the at least one cartridge (40) through the triggering means (90). The humidifier means (200) includes a container (210) configured to store water. The humidifier means (200) also includes an oxygen flow control knob (160) operatively coupled to the container (210). The oxygen flow control knob (160) is configured to control flow ratio of oxygen generated by the at least one cartridge (40). The humidifier means (200) also includes an inlet valve (220) operatively coupled to a top surface (230) of the humidifier means (200). The inlet valve (220) is configured to allow the flow of generated oxygen from the at least one cartridge (40) to the container (210). The humidifier means (200) also includes an outlet valve (240) operatively coupled to the top surface (230) of the humidifier means (200). The outlet valve (240) is configured to allow the flow of humidified oxygen generated in the container (210).

Furthermore, the at least one cartridge (40) comprising the water chamber (50), the chemical chamber (60), the holding means (80), and the triggering means (90) are substantially similar to at least two cartridges (40) comprising a water chamber (50), a chemical chamber (50), a holding means (80) and a triggering means (90) of FIG. 1. Also, the humidifier means (200) comprising the oxygen flow knob (160) is substantially similar to a humidifier (180) coupled to an oxygen flow control knob (160) as described in FIG. 1.

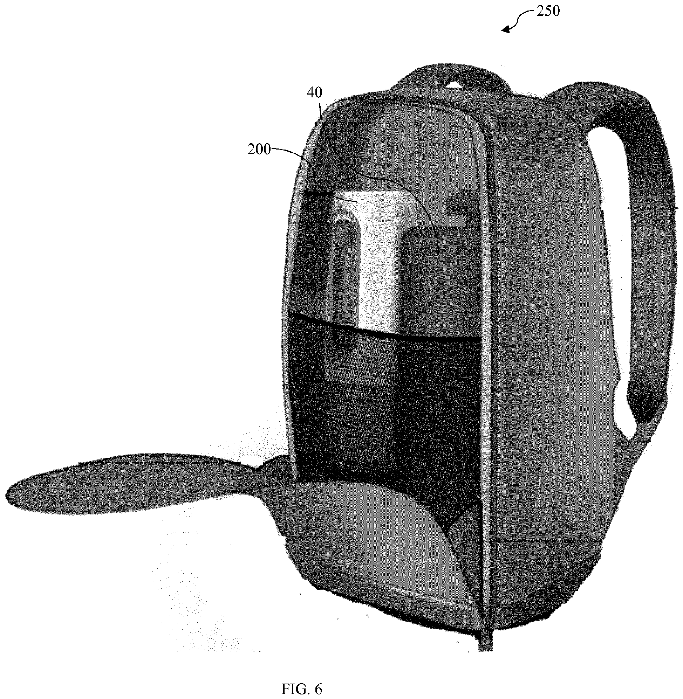

In one exemplary embodiment, the oxygen generator (190) along with the humidifier means (200) may be housed within a portable bag pack (250). The oxygen generator (40) can be attached to the humidifier means (200) through the triggering means (90) when humidification of the generated oxygen is required. The housing of the oxygen generator (40) along with the humidifier means (200) is as shown in FIG. 6.

Various embodiments of the oxygen generator to be portable as it is composed of lighter material and is compact which makes the oxygen generator more reliable. Also, as the cartridges are small and compact, replacement of the cartridges are simple and quick. In addition, as the oxygen generator includes at least two cartridges, flow of oxygen is continuous, and every alternative cartridge can be easily replaced which keeps the flow of oxygen continuous.

Furthermore, as the oxygen generator does not include the storage tank, space of the oxygen generator is reduced and thereby the flow of generated oxygen is kept continuous. In addition, the oxygen generator includes the oxygen flow control knob which gives an option for the user to choose the level or rate of oxygen required which makes the oxygen cylinder more efficient.

Also, upon triggering the top seal, the pre-defined amount of water and the one or more chemicals gets combined automatically and reacts accordingly to generate the oxygen which saves time of the user to manually combine the one or more chemicals and the pre-defined amount of water for the generation of oxygen. Due to such non-intervention of human, accuracy on the ratio of the one or more chemicals and the pre-defined amount of water is maintained thereby making the oxygen generator more accurate.

While specific language has been used to describe the disclosure, any limitations arising on account of the same are not intended. As would be apparent to a person skilled in the art, various working modifications may be made to the method in order to implement the inventive concept as taught herein.

The figures and the foregoing description give examples of embodiments. Those skilled in the art will appreciate that one or more of the described elements may well be combined into a single functional element. Alternatively, certain elements may be split into multiple functional elements. Elements from one embodiment may be added to another embodiment. For example, order of processes described herein may be changed and are not limited to the manner described herein. Moreover, the actions of any flow diagram need not be implemented in the order shown; nor do all of the acts need to be necessarily performed. Also, those acts that are not dependant on other acts may be performed in parallel with the other acts. The scope of embodiments is by no means limited by these specific examples.

## Claims

1. An oxygen generator (10) comprising:
a generator case (20) comprising at least two hollow chambers (30);
at least two cartridges (40), housed within the corresponding at least two hollow chambers (30), wherein each of the at least two cartridges (40) comprises:
a water chamber (50) configured to store a pre-defined amount of water;
a chemical chamber (60) mechanically coupled to the water chamber (50), and configured to store one or more chemicals in a pre-defined ratio,
wherein the water chamber (50) is placed above the chemical chamber (60) and is separated by a disc (70) and is pneumatically sealed;
a holding means (80) mechanically coupled to an interior top portion of the corresponding at least two cartridges (40);
a triggering means (90) mechanically held in place by the holding means (80), wherein the triggering means (90) comprises:
a top seal (100) configured to unlock a bottom seal (140) upon twisting a trigger connector (110) to a pre-defined angle;
a trigger valve (120) operatively coupled to the top seal (100), and configured to enable a process of generation of oxygen;
a hollow seal tube (130) located along a central axis of the corresponding at least two cartridges (40); and
the bottom seal (140) operatively coupled at a bottom of the hollow seal tube (130), and configured to allow the pre-defined amount of water to enter the chemical chamber (60) via a filter (150), wherein the filter (150) is fixed to the disc (70) within the chemical chamber (60),
wherein the hollow seal tube (130) is configured to:
allow flow of oxygen generated from chemical chamber (60) to the water chamber (50);
allow flow of oxygen generated in the chemical chamber (60) upon reaction of the pre-defined amount of water with the one or more chemicals to exit from the water chamber (50),
wherein the at least two cartridges (40) are placed in the corresponding at least two hollow chambers (30) of the generator case (20).

2. The oxygen generator (10) as claimed in claim 1, wherein the chemical chamber (60) of each of the at least two cartridges (40) further comprises a mechanical stirrer configured to keep the one or more chemicals in the chemical chamber (60) in constant mixing.

3. The oxygen generator (10) as claimed in claim 1, wherein the one or more chemicals comprises at least one of sodium percarbonate, potassium superoxide, peroxide species (hydrogen peroxide), urea-hydrogen peroxide and percarbamide peroxide.

4. The oxygen generator (10) as claimed in claim 1, wherein the one or more chemicals is on a form of one of a granular powder, pellets, powder in a pouch and candle stick.

5. The oxygen generator (10) as claimed in claim 1, wherein flow of oxygen in the hollow seal tube (130) is uni-directional and is controlled by the bottom seal (140).

6. The oxygen generator (10) as claimed in claim 1, wherein the trigger connector (110) is placed on a top surface of the generator case (20), and is configured to keep the connection of the at least two cartridges (40) in place during the generation of oxygen.

7. The oxygen generator (10) as claimed in claim 1, further comprising an oxygen flow control knob (160) operatively coupled to each of the at least two cartridges (40) via the corresponding trigger connector (110), and configured to control flow ratio of oxygen generated by each of the at least two cartridges (40).

8. The oxygen generator (10) as claimed in claim 7, further comprising a humidifier (180) operatively coupled to the oxygen flow control knob (160), and configured to impart humidity to the generated oxygen.

9. The oxygen generator (10) as claimed in claim 1, further comprising an oxygen level indicator (170) operatively coupled to each of the at least two cartridges (40), and configured to display oxygen level in each of the at least two cartridges (40).

10. The oxygen generator (10) as claimed in claim 1, further comprising an oxygen pressure regulator operatively coupled to each of the at least two cartridges (40), and configured to enable a uniform flow of oxygen.

11. The oxygen generator (10) as claimed in claim 1, further comprising a cooling mechanism operatively coupled to each of the at least two cartridges (40), and configured to dissipate heat from each of the at least two cartridges (40).

12. The oxygen generator (10) as claimed in claim 1, further comprising at least one storage tank operatively coupled to each of the at least two cartridges (40), and configured to enable a uniform flow of oxygen generated in the chemical chamber (60).

13. An oxygen generator (190) comprising:
at least one cartridge (40) comprising:
a water chamber (50) configured to store a pre-defined amount of water;
a chemical chamber (60) mechanically coupled to the water chamber (50), and configured to store one or more chemicals in a pre-defined ratio,
wherein the water chamber (50) is placed above the chemical chamber (60) and is separated by a disc (70) and is pneumatically sealed;
a holding means (80) mechanically coupled to an interior top portion of the corresponding at least one cartridge (40);
a triggering means (90) mechanically held in place by the holding means (80), wherein the triggering means (90) comprises:
a top seal (100) configured to unlock a bottom seal (140) upon twisting a trigger connector (110) to a pre-defined angle;
a trigger valve (120) operatively coupled to the top seal (100), and configured to enable a process of generation of oxygen;
a hollow seal tube (130) located along a central axis of the corresponding at least one cartridge (40); and
the bottom seal (140) operatively coupled at a bottom of the hollow seal tube (130), and configured to allow the pre-defined amount of water to enter the chemical chamber (60) via a filter (150), wherein the filter (150) is fixed to the disc (70) within the chemical chamber (60),
wherein the hollow seal tube (130) is configured to:
allow flow of oxygen generated from chemical chamber (60) to the water chamber (50);
allow flow of oxygen generated in the chemical chamber (60) upon reaction of the pre-defined amount of water with the one or more chemicals to exit from the water chamber;
a humidifier means (200) operatively coupled to the at least one cartridge (40) through the triggering means (90), and configured to impart humidity to the generated oxygen, wherein the humidifier means (200) comprises:
a container (210) configured to store water;
an oxygen flow control knob (160) operatively coupled to the container (210), and configured to control flow ratio of oxygen generated by the at least one cartridge (40);
an inlet valve (220) operatively coupled to a top surface (230) of the humidifier means (200), and configured to allow the flow of generated oxygen from the at least one cartridge (40) to the container (210); and
an outlet valve (240) operatively coupled to the top surface (230) of the humidifier means (200), and configured to allow the flow of humidified oxygen generated in the container (210).
